# EUROPÄISCHE PATENTANMELDUNG

(11) **EP 1 454 944 A1**
(43) Veröffentlichungstag der Anmeldung: **08.09.2004**
(21) Anmeldenummer: 04003958.8
(22) Anmeldetag: 21.02.2004
(51) Int. Cl.: C08J 5/18, A61K 9/48, C08L 29/04

(54) **Polymere Zusammensetzung auf Basis von PVA**

(30) Priorität: 03.03.2003 DE 10309064
(71) Anmelder: Clariant GmbH, 65929 Frankfurt am Main (DE)
(72) Erfinder: Huth, Hans-Ullrich, Dr., 63329 Egelsbach (DE); Rath, Heinz Jörg, Dr., 65857 Waldaschaff (DE)
(74) Vertreter: Paczkowski, Marcus, Dr.

(57) **Zusammenfassung**

Es wird eine polymere Zusammensetzung auf Basis von PVA beansprucht, die im wesentlichen besteht aus
a) 50 - 99,9 Gew.-% Polyvinylalkohol mit einem mittleren MG von 5000 - 25000 und einem Hydrolysegrad von 79 - 99,9 Mol-%, wobei der Polyvinylalkohol Carboxylgruppen oder Polyglykoleinheiten im Molekül enthalten kann, und
b) 0,1 - 50 Gew.-%, bevorzugt 0,1 - 15 Gew.-% und besonders bevorzugt 0,1 - 10 % eines oder mehrerer Polysaccharide der Formel 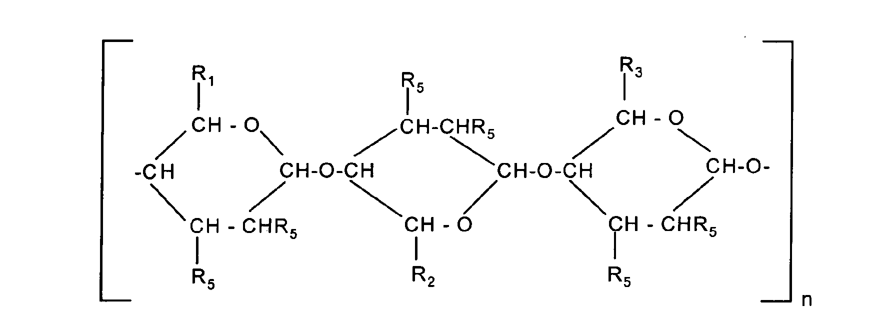
wobei
R₁, R₂, R₃ H, CH₂OH, COOMe, COOMe, COOR₄, CONHR₆ oder CH₂OSO₃Me und
Me Na, K, NH₄, Mg oder Ca bedeuten,
n eine Zahl von 20 bis 20.000, vorzugsweise von 100 bis 10.000, insbesondere von 1000 bis 9000,
R₄ C₁-C₄-Alkyl, bevorzugt Methyl,
R₅ OH, NHCOCH₃, H oder OCOCH₃ und
R₆ H, COCH₃, oder C₁-C₄, vorzugsweise Methyl bedeuten.

Diese polymere Zusammensetzung eignet sich zur Herstellung von Kapseln.

## Beschreibung

Es ist bekannt, harte und weiche Kapseln aus Gelatine herzustellen.
Gelatine ist ein Peptidgemisch mit breiter Molekulargewichtsverteilung, das durch Spaltung oder Vernetzung aus dem Tropokollagen bzw. Kollagen gebildet wird. Bedingt durch die Herkunft, durch die nichtsterilen Herstellverfahren und die Tatsache, dass Gelatine beim Erhitzen auf 120°C irreversibel chemische Eigenschaftsänderungen erfährt, muss man immer mit der Anwesenheit von Keimen (Bakterien, Hefen, Schimmelpilzen) rechnen.

Es hat nicht an Versuchen gefehlt, aufgrund dieses Infektionsrisikos durch Krankheitskeime (z.B. BSE), Gelatine durch andere natürliche oder technische Produkte wie z.B. Agar Agar, Carrageen, Carubin, Guaran, Gummi arabicum oder Zelluloseether zu ersetzen.

Polyvinylalkohol (PVA) ist ein alternatives Produkt, das in vielen wichtigen Eigenschaften der Gelatine nahe kommt. Aber auch in den toxikologischen Eigenschaften sowie bei der biologischen Abbaubarkeit ist PVA den meisten synthetischen Polymeren überlegen.
In der Herstellbarkeit und beim Preis hat PVA gegenüber Gelatine den Vorteil eines breiten Sortimentes - Molekulargewicht (MG) und Hydrolysegrad (HG) - und weil das Produkt sich sowohl als Granulat als auch als Lösung sterilisieren lässt und damit frei von Krankheitserregern herstellbar ist.
Nachteilig beim von PVA im Vergleich zu Gelatine ist die geringere Folienhärte und Foliensteifigkeit, größere Dehnbarkeit sowie eine nicht ausreichende Gallertfestigkeit (Bloom-Wert), um diesen Rohstoff ohne größere Rezeptänderung im normalen Herstellverfahren für harte und weiche Kapseln gegen Gelatine auszutauschen.

Es wurde nun gefunden, dass sich eine Mischung aus Polyvinylalkoholen und polymeren Uronsäuren oder deren Derivaten sehr vorteilhaft für die Herstellung von harten und weichen Kapseln eignet.

Gegenstand der Erfindung ist eine polymere Zusammensetzung auf Basis von PVA, bestehend im wesentlichen aus
a) 50 - 99,9 Gew.-% Polyvinylalkohol mit einem mittleren MG von 5000 - 25000, bevorzugt 10000 - 150000 und besonders bevorzugt 15000 - 100000 und einem Hydrolysegrad von 79 - 99,9 Mol-%, bevorzugt 82 - 99,9 Mol-%, besonders bevorzugt von 80 - 85, 86 - 89 und 97 - 99,9 Mol-%, wobei der Polyvinylalkohol Carboxylgruppen oder Polyglykoleinheiten im Molekül enthalten kann, und
b) 0,1 - 50 Gew.-%, bevorzugt 0,1 - 15 Gew.-% und besonders bevorzugt 0,1 - 10 % eines oder mehrerer Polysaccharide der Formel 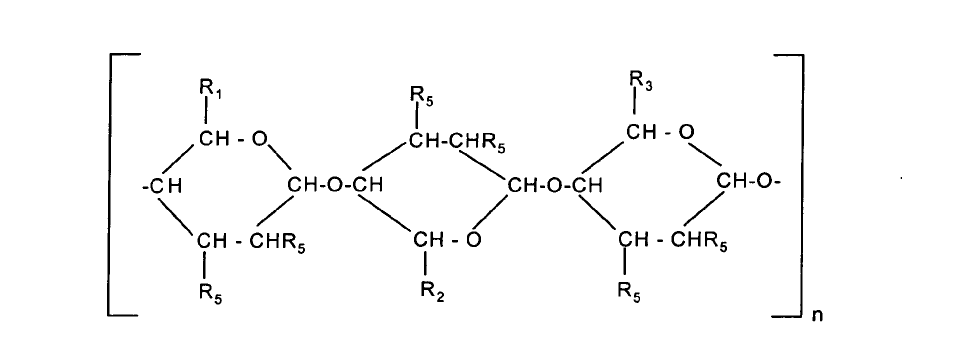
wobei
R₁, R₂, R₃ H, CH₂OH, COOH, COOMe, COOR₄, CONHR₆ oder CH₂OSO₃Me und
Me Na, K, NH₄, Mg oder Ca bedeuten,
n eine Zahl von 20 bis 20.000, vorzugsweise von 100 bis 10.000, insbesondere von 1000 bis 9000,
R₄ C₁-C₄-Alkyl, bevorzugt Methyl,
R₅ OH, NHCOCH₃, H oder OCOCH₃ und
R₆ H, COCH₃, oder C₁-C₄, vorzugsweise Methyl bedeuten.

Die erfindungsgemäßen polymeren Zusammensetzungen können zusätzlich noch 0 bis 0,5, vorzugsweise 0,01 bis 0,3 Gew.-% Entschäumer und 0 bis 10, vorzugsweise 0,01 bis 5 Gew.-% einer oberflächenaktiven Verbindung, vorzugsweise solche Verbindungen, die für den Einsatz in kosmetischen und pharmazeutischen Produkten zugelassen sind, wie z.B. Lecithin, Glycerinmono- oder -diester enthalten. Weitere mögliche Zusatzstoffe sind hydrophobierende Öle bzw. Pflanzenöle, Wachse, wie z.B. Erdnussöl, hydriertes Pflanzenöl, Kakaobutter, Sheabutter oder Bienenwachs in einer Menge von 0 bis 10, vorzugsweise 0,0001 bis 2 Gew.-%, Modifizierungs- oder Stellmittel, die für eine bessere Auflösung oder eine Trübung und Färbung der Kapsel geeignet sind, wie z.B. Kieselsäure, Eisenoxid, Calciumoxid, Talkum in einer Menge von 0 bis 3, vorzugsweise 0,1 bis 3 Gew.-% sowie übliche Weichmacher wie z.B. mehrwertige Alkohole, z.B. Polyethylenglykol, Glycerin, Sorbit oder Sorbitol in einer Menge von 0 bis 30 Gew.-%.

Bei der Herstellung der erfindungsgemäßen Zusammensetzung werden alle oben genannten Rohstoffe in der Wärme, vorzugsweise bei 40 bis 90°C hintereinander in der notwendigen Menge unter Rühren in Wasser gelöst, wobei man darauf achten sollte, dass jede einzelne Verbindung vollständig aufgelöst ist bevor die nächste folgt. Die fertige Lösung kann anschließend für 30 Minuten in einem Autoklaven sterilisiert werden und ist dann keimfrei.

### Bevorzugt geht man wie folgt vor:

Bevor man mit dem Aufheizen des Wassers beginnt, streut man die gewünschte Menge an Polysaccharid unter ausreichender Rührung ein. Anschließend heizt man das Wasser auf die für die Auflösung notwendige Temperatur, meist 80 bis 90°C, gibt dann die abgewogene Menge Polyvinylalkohol zu, wenig später die restlichen Bestandteile sowie am Schluss den Entschäumer, dabei nimmt man die Rührerdrehzahl zurück und kühlt danach ab.

Die so erhaltenen polymeren Zusammensetzungen eignen sich sehr gut für die Herstellung von wasserlöslichen Filmen, insbesondere aber für wasserlösliche Kapseln, die beispielsweise pharmazeutische Wirkstoffe oder Wirkstoffe anderer Art enthalten.

Die nachfolgenden Beispiele sollen die Erfindung erläutern ohne sie jedoch einzuschränken:

### Beispiel 1

In 180 g Wasser wurde nach dem Aufheizen unter starkem Rühren 4 g Pektin (20 % bez. auf Festsubstanz (FS) angequollen und dann 16 g PVA mit einem mittleren MG von 30 000 und einem mittleren Hydrolysegrad von 98 Mol-% (Mowiol® 4-98) so eingestreut, dass das Granulat nicht verklumpt. Man wartete bis eine fast klare Lösung entstanden war. Dann wurden als Emulgator 0,4 g Lecithin 63 % und als Entschäumer 0,1 g C₁₀/C₂₂-Alkohol-EO/PO-Addukt (Genapol® 2822) zugegeben. Wenn alles gelöst war, ließ man abkühlen. Man erhielt eine Lösung mit einem Feststoffgehalt von 9,9 Gew.-% und einer Viskosität von 836 mPas bei 20°C im Brookfield Viskosimeter LVT (3/30).

### Beispiel 2

In 180 g Wasser wurden unter starkem Rühren und Aufheizen 6 g Gummi arabicum (30 % bez. auf FS) gelöst und dann 14 g PVA mit einem mittleren MG von 31 000 und einem mittleren Hydrolysegrad von 88 Mol-% (Mowiol® 4-88) so eingestreut, dass das Granulat nicht verklumpt. Nachdem eine fast klare Lösung entstanden war, fügte man anschließend 0,5 g CaCO₃, 2 g Sheabutter und 0,9 g Lamepon S (Fa. Spinnrad), einzeln und jeweils nach dem Auflösen bzw. Verteilen der vorherigen Substanz zu. Nachdem der Schaum zerfallen ist, kühlte man ab. Die leicht trübe Lösung wies einen Feststoff von 8,6 % und eine Viskosität von 640 mPas bei 20°C im Brookfield Viskosimeter LVT (3/30) auf.

### Beispiel 3

180 g Wasser wurde aufgeheizt und 1 g Na-Alginat (5 % bez. auf FS) unter starkem Rühren angequollen. Danach wurden 19 g PVA mit einem mittleren MG von 47 000 und einem mittleren Hydrolysegrad von 98 Mol-% (Mowiol 6-98) so eingestreut, dass das Granulat nicht verklumpt. Man wartete bis eine fast klare Lösung entstanden war. Nach dem Abkühlen wurde das verdampfte Wasser ergänzt. Die fertige, leicht schaumige Lösung hatte eine schwach bräunliche Farbe und besaß einen Feststoffgehalt von 9,9 %. Die gemessene Viskosität bei 20°C betrug nach Brookfield LVT (3/60) 240 mPas.

### Beispiel 4

180 g Wasser wurde aufgeheizt und unter starkem Rühren fügte man 2 g Pektin (10 % bez. auf F S) zu und wartete bis eine fast klare Lösung entstanden war. Danach wurden 18 g PVA mit einem mittleren MG von 150 000 und einem mittleren Hydrolysegrad von 88 Mol-% (Mowiol 23-88) so eingestreut, dass das Granulat nicht verklumpt. Nachdem man mit dem Auflösen fertig war, werden als Emulgator 0,4 g Lamepon (Fa. Spinnrad) zugegeben. Verdampftes Wasser wurde nach dem Abkühlen ergänzt. Die Lösung, die einen Feststoffgehalt von 10 Gew.-% aufwies, ergab im Brookfield Viskosimeter LVT (3/30) eine Viskosität von 940 mPas bei 20°C.

### Beispiel 5 (Vergleichsbeispiel)

Man ging wie in Beispiel 1 beschrieben vor, nahm 160 g Wasser und 40 g PVA, ließ Pektin, Emulgator und Entschäumer weg und erhielt eine 20,6 % farblose, klare Lösung und einer Viskosität von rd. 1620 mPas bei 20°C mit einem Brookfield Viskosimeter LVT (3/30).

Die unter 1 - 5 hergestellten Lösungen wurden alle auf einen Feststoffgehalt von 10 Gew.-% gebracht und mit einer 1500 µ Rakel auf eine Polyesterfolie aufgerakelt und bei Raumtemperatur für 3 Tage staubfrei getrocknet. Anschließend wurden daraus 6 x 6 cm große quadratische Stücke geschnitten und in einen Metallrahmen eingespannt. Die Metallrahmen wurden in ein 500 ml Becherglas mit Wasser und Thermometer bei unterschiedlicher Temperatur gehängt und mit einem Magnetrührer bei mäßiger Geschwindigkeit gerührt. Danach wurde die Zeit bestimmt, bis zu der sich die Polymerfolie aufgelöst hatte bzw. zerfallen war.

### Ergebnis:

Auflösungszeit für die polymeren Zusammensetzungen gemäß den Beispielen 1 bis 5 in Minuten

| | Beispiel 1 | Beispiel 2 | Beispiel 3 | Beispiel 4 | Beispiel 5 |
|---|---|---|---|---|---|
| Temperatur 40°C | Folie zerfällt nach 6 Min. | Folie löst sich nach 1 Min. | Folie reißt in große Stücke nach 6 Min. | Folie löst sich nach 40 Sek. auf | unlöslich, Folie reißt nach 25 Min. ein |
| Temperatur 60°C | Folie zerfällt nach 3 Min. | Folie löst sich nach 1 Min. | Folie zerfällt, löst sich nach 3 Min. | Folie löst sich nach 3 Sek. auf | unlöslich, Folie reißt nach 18 Min. ein |

Aus den gleichen Filmen wurden Prüfkörper zur Bestimmung der Reißfestigkeit nach DIN ISO 527 ausgestanzt und 7 Tage bei 23°C und 50 % rel. Luftfeuchtigkeit gelagert. Anschließend wurde die Reißfestigkeit bei einer Zuggeschwindigkeit von 300 mm/ Min. bestimmt.

### Ergebnis:

Reißfestigkeit für die polymeren Zusammensetzungen gemäß den Beispielen 1 bis 5:

| Beispiel 1 | Beispiel 2 | Beispiel 3 | Beispiel 4 | Beispiel 5 |
|---|---|---|---|---|
| 73,8 | 16,0 | 39,8 | 78,4 | 24,2 |

## Patentansprüche

1. Polymere Zusammensetzung auf Basis von PVA, bestehend im wesentlichen aus
a) 50 - 99,9 Gew.-% Polyvinylalkohol mit einem mittleren MG von 5000 - 25000 und einem Hydrolysegrad von 79 - 99,9 Mol-%, wobei der Polyvinylalkohol Carboxylgruppen oder Polyglykoleinheiten im Molekül enthalten kann, und
b) 0,1 - 50 Gew.-%, bevorzugt 0,1 - 15 Gew.-% und besonders bevorzugt 0,1 - 10 % eines oder mehrerer Polysaccharide der Formel 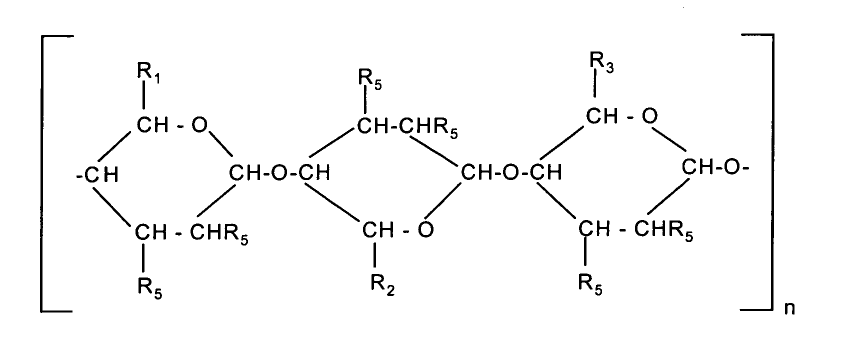
wobei
R₁, R₂, R₃ H, CH₂OH, COOH, COOMe, COOR₄, CONHR₆ oder CH₂OSO₃Me und
Me Na, K, NH₄, Mg oder Ca bedeuten,
n eine Zahl von 20 bis 20.000, vorzugsweise von 100 bis 10.000, insbesondere von 1000 bis 9000,
R₄ C₁-C₄-Alkyl, bevorzugt Methyl,
R₅ OH, NHCOCH₃, H oder OCOCH₃ und
R₆ H, COCH₃, oder C₁-C₄, vorzugsweise Methyl bedeuten.

2. Polymere Zusammensetzung nach Anspruch 1, **dadurch gekennzeichnet, dass** sie zusätzlich 0 bis 5 Gew.-% Entschäumer enthält.

3. Polymere Zusammensetzung nach Anspruch 1, **dadurch gekennzeichnet, dass** sie zusätzlich 0 bis 10 Gew.-% einer oberflächenaktiven Verbindung enthält.

4. Polymere Zusammensetzung nach Anspruch 1, **dadurch gekennzeichnet, dass** sie 0 bis 10 Gew.-% hydrophobierende Öle enthält.

5. Polymere Zusammensetzung nach Anspruch 1, **dadurch gekennzeichnet, dass** sie 0 bis 3 Gew.-% Modifizierungs- oder Stellmittel enthält.

6. Polymere Zusammensetzung nach Anspruch 1, **dadurch gekennzeichnet, dass** sie 0 bis 30 Gew.-% Weichmacher enthalten.

7. Verwendung der polymeren Zusammensetzungen nach Anspruch 1 zur Herstellung von wasserunlöslichen Kapseln oder Filmen.
